# EUROPEAN PATENT APPLICATION

(11) **EP 4 760 745 A1**
(43) Date of publication of application: **17.06.2026**
(21) Application number: 25216288.8
(22) Date of filing: 17.11.2025
(51) Int. Cl.: G16H 50/20, G16H 50/70, A61B 5/00, G16H 30/40

(54) **AUTOMATED TAGGING OF PARAMETER WAVEFORMS**

(30) Priority: 10.12.2024 US 202418976119
(71) Applicant: GE Precision Healthcare LLC, Waukesha, WI 53188 (US)
(72) Inventor: MISHRA, Isani, 560066 Bengaluru (IN); RAGHAVAN, Jayaram, 560066 Bengaluru (IN); PARDASANI, Rohit, 560066 Bengaluru (IN)
(74) Representative: Kilburn & Strode LLP

(57) **Abstract**

A waveform anomaly detection system (360) is provided that automatically detects and tags waveform data patterns in physiological patient (time-series) data that match predefined, clinically validated patterns (1300, 1400, 1500), to enable clinicians to match and corroborate their own observations with tool-triggered findings. As a result, clinicians may make more informed clinical decisions without having to manually review enormous amounts of time-series data. Additionally, when anomalous waveform patterns (1300, 1400, 1500) are detected, relevant patient data from an electronic medical record (EMR) (350) or similar databases and/or platforms (352) may be automatically retrieved and integrated into the waveform data, such that the integrated data may be viewed together on a display device (140). The data may be stored with the anomalous waveform patterns (1300, 1400, 1500) in a database (354) of the waveform anomaly detection system (360), where analysis results can be accessed conveniently by a caregiver of the patient, for example, using a personal computing device such as a smart phone (140).

## Description

### TECHNICAL FIELD

Embodiments of the subject matter disclosed herein relate to patient monitoring, and in particular to monitoring of physiological waveform data of patients.

### BACKGROUND

Patients monitored in critical care units (CCU) may be kept under surveillance with continuous monitoring of their physiological parameters, including high fidelity waveform data and numerical parameter data, for several days. This generates huge amounts of time-series patient physiological data collected over the CCU stay. However, clinicians may only be notified of the device-triggered alarm events, for example, where deviations are detected in the numerical parameter data. As a result, clinicians may have to scan through large amounts of the waveform data to identify any changes indicative of prevailing clinical or sub-clinical conditions. This manual scan is cumbersome and time consuming, and events can go unnoticed due to human error.

### SUMMARY

In one example, the problems described above may be addressed by a waveform anomaly detection system, comprising a processor, and a memory storing instructions that when executed, cause the processor to receive physiological patient waveform data acquired via a sensor positioned on a body of a patient; detect an anomalous pattern in a portion of the physiological patient waveform data in real time as the physiological patient waveform data is received; retrieve clinical data relating to the anomalous pattern of the patient from one or more electronic medical record (EMR) systems; store the retrieved clinical data together with the portion of the physiological patient waveform data in the memory; and display the portion of the physiological patient waveform data having the anomalous pattern on a display device together with the retrieved clinical data and/or a link to the retrieved clinical data; wherein detecting the anomalous pattern in the portion of the physiological patient waveform data further comprises comparing the portion with a plurality of stored templates of anomalous patterns of waveform data stored in a waveform signature database in the memory, the templates generated from examples of anomalies included in standard medical literature that are validated and firmly established in clinical practice. When the anomalous pattern is detected, a notification may be sent to a caregiver of the patient (e.g., to a smart phone of the caregiver) and the portion of the physiological patient waveform data having the anomalous pattern may be displayed on the display device along with the retrieved clinical data and/or the link to the retrieved clinical data in response to the caregiver opening the notification. The physiological patient waveform data may include electrocardiogram (ECG) waveform data, pleth waveform data acquired via a pulse oximeter, arterial waveform data, end-tidal carbon dioxide (ETCO2) waveform data acquired via a capnography system, or a different type of time-series data. The retrieved clinical data may include, for example, demographic data, lab data, medication data, clinical notes, and/or other physiological parameter data of the patient.

In various examples, the waveform signature database may be populated by retrieving and digitizing images of anomalous physiological patient waveform data taken from established medical literature using a plot digitizer. The digitized images may be resampled prior to storing in the waveform signature database. Additionally or alternatively, images of new patterns encountered by a user of the waveform anomaly detection system may be retrieved, digitized, and included in the waveform signature database.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a pictorial view of an exemplary patient monitoring system;
FIG. 2 shows an exemplary placement of sensors of the patient monitoring system on a body of a patient;
FIG. 3 is a schematic diagram of an exemplary waveform anomaly detection system used in conjunction with the patient monitoring system;
FIG. 4 is a plot of physiological patient waveform data including a plurality of heartbeats, as prior art;
FIG. 5 is a flowchart illustrating an exemplary method for detecting anomalous patterns in physiological patient waveform data;
FIG. 6 is a flowchart illustrating an exemplary method for matching physiological patient waveform data with an anomalous waveform pattern stored in a database;
FIG. 7 is a flowchart illustrating an exemplary method for populating database of a waveform anomaly detection system with a plurality of examples of anomalous physiological patient waveform data;
FIG. 8 shows a first plot of capnographic waveform data of a healthy patient compared with a second plot of capnographic waveform data of a patient suffering a bronchospasm;
FIG. 9 shows a graph illustrating distances between capnographic waveform data of a healthy patient and two different waveform patterns;
FIG. 10 shows a graph illustrating distances between capnographic waveform data of a patient suffering a bronchospasm and two different waveform patterns;
FIG. 11 shows a first plot of a capnographic waveform having a first sampling rate, and a second plot of the capnographic waveform resampled at a second sampling rate;
FIG. 12 shows an exemplary plot of physiological patient waveform data of a patient displayed on a display device along with related clinical data of the patient;
FIG. 13 shows a first plot of capnographic waveform data of a patient suffering from shock, as prior art;
FIG. 14 shows a second plot of capnographic waveform data of a patient suffering from hypoxia, as prior art; and
FIG. 15 shows a third plot of capnographic waveform data of a patient suffering from emphysema, as prior art.

### DETAILED DESCRIPTION

This description and embodiments of the subject matter disclosed herein relate to analyzing waveforms in physiological patient waveform data, meaning, time-series data acquired over regular time intervals via electrophysiological monitoring. The phrase "time-series data" can refer to a sequence of data that is repeatedly generated and/or captured by a device (e.g., a computing device) at a plurality of time values during a certain time interval. Examples of time-series data include the continuous monitoring of a person's heart rate, hourly readings of air temperature, monthly rainfall data, and the like. Time-series data can be analyzed to understand the underlying structures and functions that produce the observations. Understanding the mechanisms of time-series data allows a mathematical model to be developed that explains the data in such a way that prediction, monitoring, or control can occur.

Electrophysiological monitoring is a common way to assess the health of a patient and to diagnose and/or detect the presence of an adverse health condition based on physiological time-series data. For example, an electrocardiograph (ECG) generates waveform data used to assess cardiac health, and an electroencephalograph (EEG) generates waveform data used to assess brain health. Arterial waveform data can be used to diagnose arteriosclerosis, cardiac disorders, and/or damaged heart valves. Capnography waveform data can be used to diagnose shock.

Sometimes the results of electrophysiological monitoring produce results that indicate anomalies or abnormalities. In some cases, the anomalies may trigger alerts in embedded software programs that monitor the waveform data. However, in other cases, the anomalies may include useful diagnostic information, but may not meet criteria for any identifiable condition. Such results are typically considered inconclusive or ambiguous. In such situations, a clinician may be unable to glean any useful information from the waveform data and may disregard the results of the physiological monitoring as inconclusive and/or not providing any indication regarding patient health or diagnosis. Additionally, a lack of embedded pattern recognition algorithms in the medical devices leads to large amount of anomalous waveform patterns going unidentified. The clinician may perform a periodic review to manually scan the time-series data while making clinical decisions about their patient's treatment management, but such reviews may be time-consuming, cumbersome, and prone to missing events of interest in the waveform data.

To address this problem, a waveform anomaly detection system is provided herein to automatically detect and tag waveform data patterns in the collected time-series data that match predefined, clinically validated patterns, to enable clinicians to match and corroborate their own observations with tool-triggered findings. As a result, clinicians may be empowered to make more informed clinical decisions without having to manually review enormous amounts of time-series data. Additionally, when anomalous waveform patterns are detected, relevant patient data from an electronic medical record (EMR) or similar databases and/or platforms may be automatically retrieved and integrated into the waveform data, such that the integrated data may be viewed together on a display device.

For example, a pattern in ECG data may indicate a first condition, and the waveform anomaly detection system may search for clinical patient data relating to the first condition (e.g., demographic data, medical labs, medical history, medications, etc.) in the EMR and/or other data platforms. The retrieved data may be analyzed, and may suggest or reveal a second condition, and the waveform anomaly detection system may search for clinical patient data relating to the second condition (e.g., demographic data, medical labs, medical history, medications, etc.) in the EMR and/or other data platforms. In this way, a broad set of patient data relevant to the detected anomaly may be retrieved. The data may be stored with the anomalous waveform patterns in a database of the waveform anomaly detection system, where analysis results can be accessed conveniently by a caregiver of the patient, for example, using a personal computing device such as a smart phone. Timely alerts may also be generated via an app on the smart phone. Such a system could be a powerful aid for clinicians in ensuring that aberrations or abnormalities in real-time continuously monitored physiological data gets proactively identified by an intelligent algorithm to enable informed clinical decision-making and patient treatment management.

Further, clinicians in their early learning phase in clinical practice may depend on identifying parameter numeric violations, which may be called out in alerts generated by embedded patient monitoring programs. These clinicians currently do not typically trace back to the corresponding waveform pattern indicative of the clinical condition. Therefore, the system described herein may also serve as a training tool for clinicians.

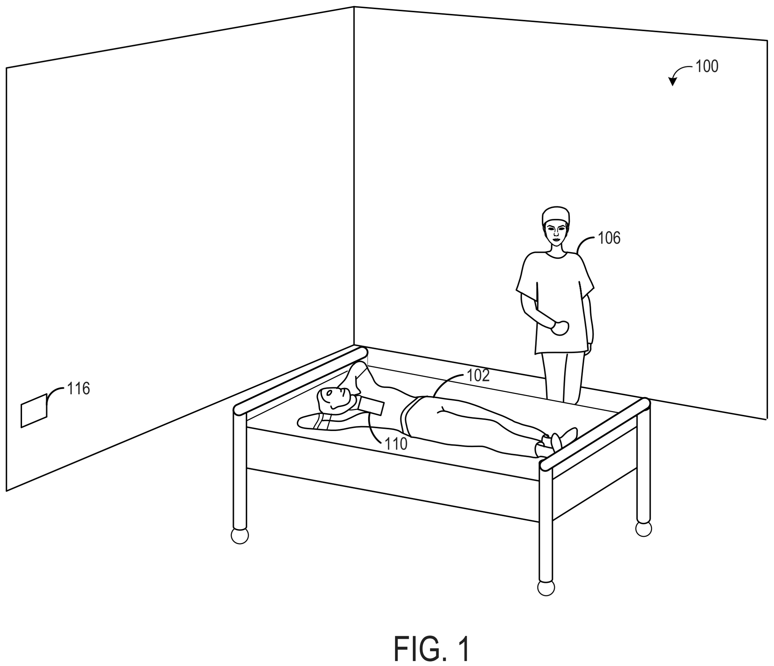

FIG. 1 shows an example patient monitoring environment 100, which in the depicted example is a patient room in a hospital or other medical facility. The patient monitoring environment 100 may include one or more patient monitoring devices, monitoring one or more physiological parameters. The patient monitoring environment 100 includes a patient 102 being monitored by one or more monitoring devices and also being attended to by a clinician 106. Clinician 106 may be a nurse, physician, medical technologist, or another suitable medical professional. The monitoring devices include a patient monitor 110. The patient monitoring environment 100 depicts the use of one monitoring device but it is understood this is non-limiting as the patient monitoring environment 100 could include one device monitoring one parameter, one device monitoring more than one parameter, multiple devices each monitoring one or more than one parameter, and so on. Due to differing patient conditions and the varying patient monitoring needs, one or more devices may be used to support the monitoring needs of the patient and capable of supporting monitoring in various conditions (e.g., in room, in transport, patient ambulation).

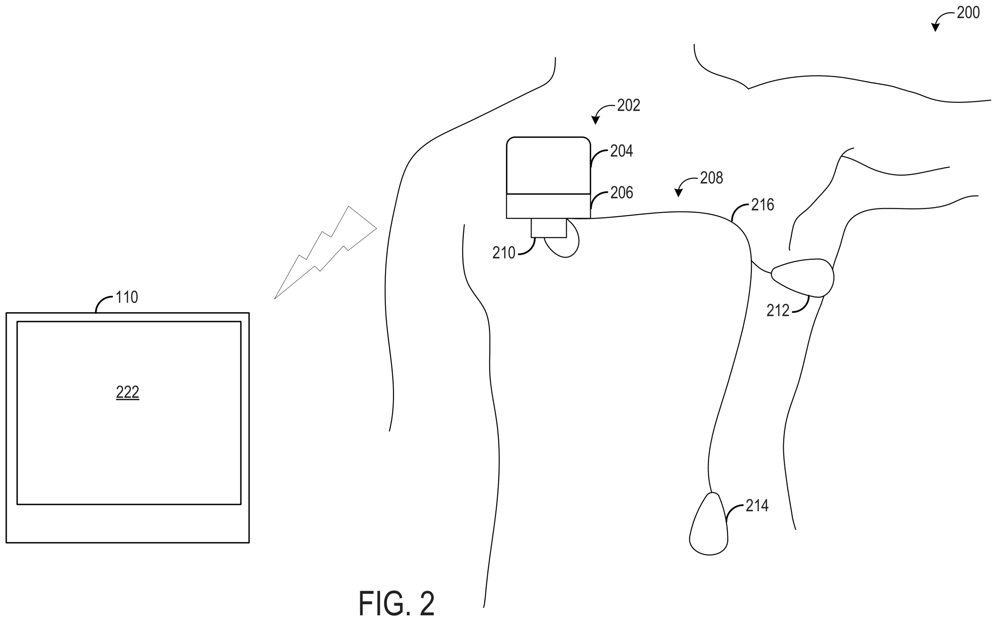

Patient monitor 110 may include one or more telemetry devices (with different sensor capabilities) housed in a common module or housed in two or more separate modules. Patient monitor 110 may be positioned on the patient (e.g., via a pouch, holder, or similar, attached to a belt of the patient) or on a movable module (e.g., a wheeled module), such that patient monitor 110 may leave the patient room if the patient leaves the patient room, and may travel with the patient. Patient monitor 110 may be connected to the patient 102 via one or more leads or other components or in wireless communication with an associated sensor (an example of which is shown in FIG. 2), in order to monitor one or more parameters of the patient (such as ECG, respiration, blood oxygen level, etc.).

The patient monitoring data collected by patient monitor 110 may be sent wirelessly (e.g., via WiFi, Bluetooth, Medical Body Area Network (MBAN)) and/or via a hardwired networked connection to one or more associated devices for processing, analysis, storage, display, etc., such as a central station, patient monitoring database, and/or different patient monitoring system. The methods of wireless communication used by patient monitor 110 and the receiving systems vary widely based on the technology used. In one example communication approach, to facilitate the transfer of the patient monitoring data collected by patient monitor 110, patient monitoring environment 100 and nearby areas (e.g., hallways, closets, open spaces) may include one or more access points 116. Access point 116 may receive and send information (e.g., wirelessly) to patient monitor 110 (e.g., the patient monitoring data, communication status). The access point 116 sends the received information to a processing server, a central station, a telemetry monitoring system, and/or another suitable device. If patient 102 leaves the patient room and moves throughout the medical facility, patient monitoring data collected by patient monitor 110 may be sent to other access points located throughout the medical facility. It is understood that this example using a transceiver and access point 116 for data communications is one of many different technologies suitable for sharing acquired patient monitoring data with the associated data processing, analysis, storage, and information viewing system components and infrastructure.

FIG. 2 shows an example patient monitoring system 200 including a sensor system 202. Sensor system 202 includes a host device 204, a sensor device 206, and a sensor 208. As shown in FIG. 2, the sensor device 206 is mechanically connected to the host device 204 and the sensor 208 is mechanically connected to the sensor device 206.

The sensor system 202 is in the form of a respiration rate monitor, and thus the sensor 208 includes a plurality of electrodes, including electrode 212 and electrode 214, each of which are connected to a sensor base/connector 210 via a cable harness 216 (also referred to as a cable). While not shown in FIG. 2, in some examples, an electrode and/or adhesive patch of the sensor 208 may be positioned behind the host device 204 and/or sensor device 206. The electrodes (and adhesive patch) are shown as being coupled to a chest of a patient.

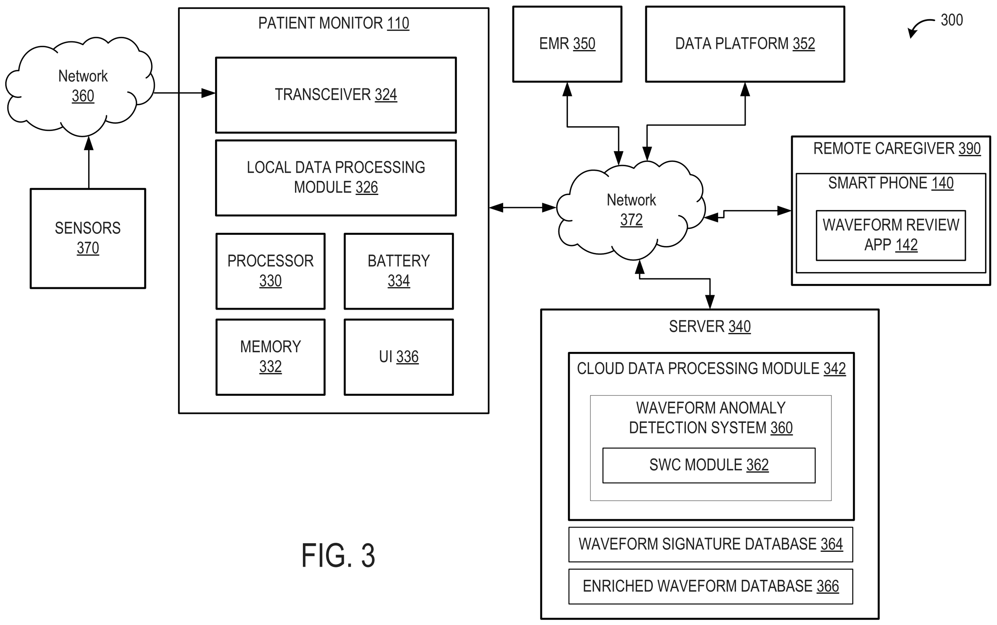

The host device 204 is configured to communicate wirelessly with the patient monitor 110 and/or a processing system. In some examples, the host device 204 may communicate with the processing system (not shown in FIG. 2; an example processing system is shown in FIG. 3) via the patient monitor 110. The patient monitor 110 may include a display screen 222, via which physiological data collected by sensor 208 may be displayed to a user and/or via which notifications may be displayed to a user.

Referring now to FIG. 3, a patient monitoring system 300 is shown that may be a non-limiting example of the patient monitoring system 200 described with respect to FIG. 2. The patient monitoring system 300 includes the patient monitor 110 of FIG. 1, which may be connected to one or more sensors 370, where patient data may be transmitted from the one or more sensors 370 to patient monitor 110 over a network 372. Patient monitor 110 may also send data to a server 340, and receive data transmitted from server 340 over the network 372. In various embodiments, the patient monitoring system 300 may be established within a hospital environment or healthcare facility, such as within a patient monitoring environment such as the patient monitoring environment 100 of FIG. 1.

The one or more sensors 370 may be specially designed devices for sensing a certain type or types of patient data via placement on a patient's body, and communicating the patient data to patient monitor 110. The one or more sensors 370 may further include a plurality of sensors of different types or the same type. The one or more sensors 370 may include sensors for obtaining physiological patient data from a patient. For example, the one or more sensors may include, but are not limited to, a 3-lead ECG, a pulse oximetry sensor, a blood pressure sensor, a digital stethoscope, a respiratory sensor, a temperature sensor, and the like. The one or more sensors 370 may include a combination of one or more different kinds of sensor. One example of the sensors 370 is the sensor 208 described with respect to FIG. 2.

The physiological patient data is alternatively referred to herein as patient data. The patient data may include, for example, vital signs of the patient, such as a blood pressure or a pulse, and/or any other type of data that may be acquired from a sensor capable of acquiring patient physiological patient data in real-time. Thus, the data sensed by the one or more sensors 370 correlates to the type of sensors in one or more sensors 370 and may include ECG data, PPG data, blood pressure data, SpO2 data, respiratory rate, otoscope data, temperature data, and the like. It will be appreciated that the types of sensors listed above are mentioned for illustrative purposes, and the one or more sensors 370 may additionally include other types of sensors for obtaining physiological patient data of a patient without departing from the scope of this disclosure.

The one or more sensors 370 may communicate the acquired patient data wirelessly to patient monitor 110 over the network 372, which may include in a non-limiting manner, a wide area network (WAN); a local area network (LAN); the Internet; a wired or wireless (e.g. optical, Bluetooth, Bluetooth Low Energy (BLE), radio frequency (RF)) network; a cloud-based computer infrastructure of computers, routers, servers, gateways, etc., or any combination thereof associated therewith that allows one or more computing devices within the patient monitoring system 300 to connect with each other. The network 372 may be or include a public network, or a private network associated with a portion of a care facility, for example a surgery module or department of a hospital, or may be more broadly located across medical devices of an entire hospital or hospital system. In some embodiments, patient monitor 110 and the one or more sensors 370 may be communicatively coupled via a wireless personal area network (PAN) technology such as MBAN. In other embodiments, any PAN technology may be used, such as induction wireless, infrared wireless, ultra wideband (UWB), Bluetooth^{®}, or any other similar technology for wireless communication between co-located devices. For example, the one or more sensors 370 may communicate with patient monitor 110 via an MBAN network of network 372, and patient monitor 110 may communicate with other elements of patient monitoring system 300 via a WiFi network.

Patient monitor 110 may include a transceiver 324, a local data processing module 326, a processor 330, a memory 332, a battery 334, and a user interface (UI) 336. Patient monitor 110 may be adapted to receive data over the network 372 via the transceiver 324. The transceiver 324 may also be configured to transmit data over the network 372. In some embodiments, the transceiver 324 may be or may include a WLAN wireless card. In some embodiments, the WLAN card may be an original equipment manufacturer (OEM) card, and may include a storage medium having computer executable code and a processor to execute that code, thus effectuating the operation of the WLAN card. Patient monitor 110 may use the transceiver 324 to connect to the network 372. For example, the transceiver 324 may connect to the network via an access point of the network (e.g., access point 116 of FIG. 1) arranged at a location of the hospital environment, for example, in proximity to patients being monitored at a care module. The network 372 may receive wirelessly transmitted information from the access point, and relay the information to one or more connected devices and/or a hospital information system suitable for collecting and managing such information.

Patient monitor 110 may include a processor 330. The processor 330 may control the operation of patient monitor 110 in response to control signals from the UI 336. In some embodiments, the UI 336 may be integrated into patient monitor 110, where a user may interact with, adjust, or select control elements in the UI 336 (e.g., buttons, knobs, touchscreen elements, etc.) to send one or more control signals to the processor 330 from the UI 336. In other embodiments, the UI 336 is not integrated into patient monitor 110, and the user may interact with, adjust, or select control elements in UI 336 via a user input device, such as a mouse, track ball, touchpad, etc., or the operator may interact with UI 336 via a separate touchscreen, where the operator touches a display screen of UI 336 to interact with UI 336, or via another type of input device.

The UI 336 may include a display (e.g., screen or monitor) and/or other subsystems. Patient monitor 110 may be in the form of a laptop computing device, a tablet, a smart phone, or any other device configured to transmit data over a network. For example, a patient receiving remote care from their home may use an iPhone or iPad as patient monitor 110.

The processor 330 may execute instructions stored on a memory 332 to control patient monitor 110. As discussed herein, the memory 332 may include any non-transitory computer readable medium in which programming instructions are stored. For the purposes of this disclosure, the term "tangible computer readable medium" is expressly defined to include any type of computer readable storage. The example methods and systems may be implemented using coded instruction (e.g., computer readable instructions) stored on a non-transitory computer readable medium such as a flash memory, a read-only memory (ROM), a random-access memory (RAM), a cache, or any other storage media in which information is stored for any duration (e.g. for extended period time periods, permanently, brief instances, for temporarily buffering, and/or for caching of the information). Computer memory of computer readable storage mediums as referenced herein may include volatile and non-volatile or removable and non-removable media for a storage of electronic-formatted information such as computer readable program instructions or modules of computer readable program instructions, data, etc. that may be stand-alone or as part of a computing device. Examples of computer memory may include any other medium which can be used to store the desired electronic format of information and which can be accessed by the processor or processors or at least a portion of a computing device. In various embodiments, the memory 332 may include an SD memory card, an internal and/or external hard disk, USB memory device, or similar modular memory.

Program code embodied on a computer readable medium may be transmitted using any appropriate medium, including but not limited to wireless, wireline, optical fiber cable, RF, etc., or any suitable combination of the foregoing. Computer program code for carrying out operations for aspects of the present system may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Upon being received at patient monitor 110, the patient data may be processed by local data processing module 326. Processing the patient data may include, for example, comparing values of the patient data with one or more threshold values stored in the memory 332. For example, patient monitor 110 may include one or more lookup tables stored in the memory 332 with the one or more threshold values. During processing of the patient data, a threshold value of the one or more threshold values may be retrieved from the one or more lookup tables and compared to a corresponding value of the patient data. For example, if a blood pressure of the patient exceeds a threshold blood pressure (e.g., retrieved from the one or more lookup tables), a health status alert may be generated. In response to the health status alert being generated, an alarm may be generated, to be displayed to a caregiver at a display of the UI 336.

In some embodiments, the patient data and/or results of any processing of the patient data (e.g., health status information) may be transmitted over the network 372 to server 340. Some or all of the processing of the patient data may be carried out at server 340. For example, under some conditions, the patient data received at patient monitor 110 from the one or more sensors 370 may be transmitted to server 340 over the network 372 without being processed at local data processing module 326, and the patient data may be processed at a cloud data processing module 342 of server 340.

In particular, cloud data processing module 342 may include a waveform anomaly detection system 360, which may process physiological patient waveform data acquired via patient monitor 110. The waveform data may include, for example, fetal monitor data, electrocardiogram (ECG) data, electroencephalogram (EEG) data, intensive care unit (ICU) patient monitoring data, electromyography (EMG) data, and/or other types of waveform data. The waveform data may include a plurality of data values generated by patient monitor 110. The data values may be measured by patient monitor 110 at periodic intervals (e.g., once per second, once per minute, etc.), and transmitted to one or more listening or display devices in addition to waveform anomaly detection system 360. For example, in one embodiment, patient monitor 110 is a heart rate monitor that monitors a heart rate of a patient in a hospital, and transmits the heart rate to waveform anomaly detection system 360 as a continuous stream of data. A caregiver of the patient may view the heart rate via UI 336. In some embodiments, the measured data values may be streamed wirelessly to waveform anomaly detection system 360 via network 372. However, in other embodiments, the data values may be transmitted to waveform anomaly detection system 360 via a wired connection.

As described in greater detail below, waveform anomaly detection system 360 may include a signature and waveform comparator (SWC) module 362 configured to compare the physiological patient waveform data with a plurality of anomalous waveform pattern templates stored in a waveform signature database 364. By comparing the physiological patient waveform data with the plurality of abnormal waveform pattern templates, SWC module 362 may detect anomalous patterns in the physiological patient waveform data. When an anomalous pattern is detected, SWC module 362 may retrieve data relating to the anomalous pattern from an EMR 350 of the health care system and/or one or more additional data platforms 352 that collect and/or store patient data. The retrieved data may be stored with the detected anomalous pattern in an enriched waveform database 366, and/or displayed to a caregiver via UI 336.

Server 340 may also serve the patient data and/or results to a wireless device of a remote caregiver 390, such as a smart phone 140, so that the remote caregiver 390 may view the patient data and/or results. In particular, remote caregiver 390 may view physiological patient waveform data processed by a waveform anomaly detection system 360. For example, remote caregiver 390 may be notified by the waveform anomaly detection system 360 that an abnormal waveform pattern was detected in a patient of remote caregiver 390. Remote caregiver 390 may view the notification in a waveform review app 142 installed on smart phone 140. Waveform review app 142 may display the abnormal waveform pattern of the patient, and the retrieved clinical data (including other waveform data) or links to the retrieved clinical data may be displayed concurrently with the abnormal waveform pattern.

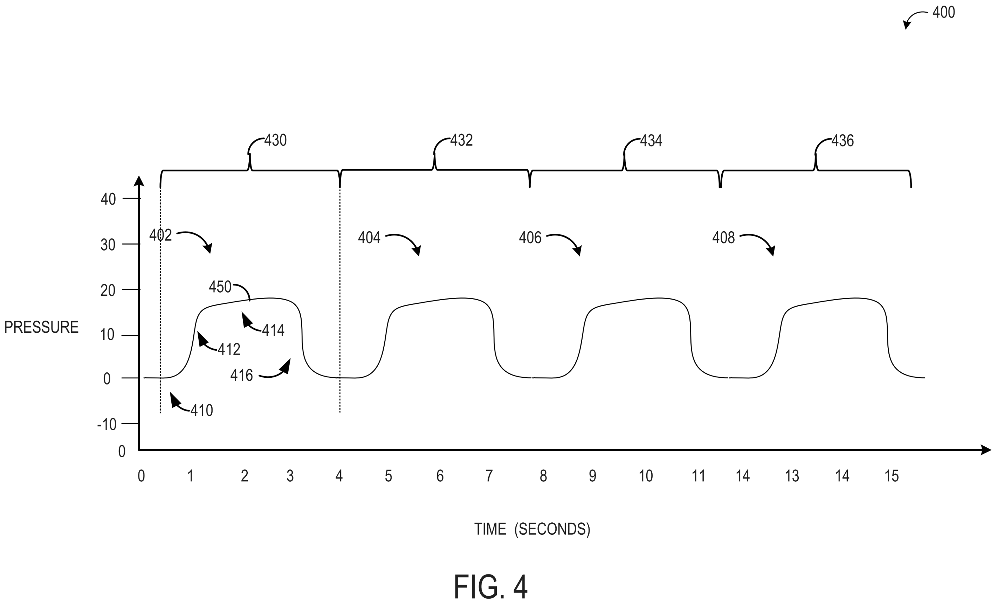

FIG. 4 shows exemplary waveform data of a patient that may be analyzed by waveform anomaly detection system 360, as prior art. FIG. 4 shows capnography time-series data 400 of a patient detected by a monitoring device that detects a partial pressure of CO₂ in an airway of a patient, which may be an example of patient monitor 110 of FIG. 3.

Capnography time-series data 400 shows a plot 450 that includes a plurality of waveforms, where each waveform of the plurality of waveforms begins and ends with at a respiratory baseline 410. Each waveform includes three portions: an expiratory upstroke 412, an expiratory plateau 414, and an inspiratory downstroke 416, ending at respiratory baseline 410. A first waveform 402 has a first duration 430; a second waveform 404 has a second duration 432; a third waveform 406 has a third duration 434; and a fourth waveform 408 has a fourth duration 436.

An end-tidal carbon dioxide (ETCO2) measurement may be taken at an end of the expiratory plateau 414 of each waveform. The ETCO2 measurement may be used, for example, by a software program embedded in the monitoring device or in a cloud to assess a condition of the patient. For example, if the ETCO2 measurement decreases below a threshold measurement, an alert may be triggered. However, while the ETCO2 measurement alone may not be sufficient to indicate the condition of the patient, the condition of the patient may be determined by a shape and/or trend in waveforms 402-408.

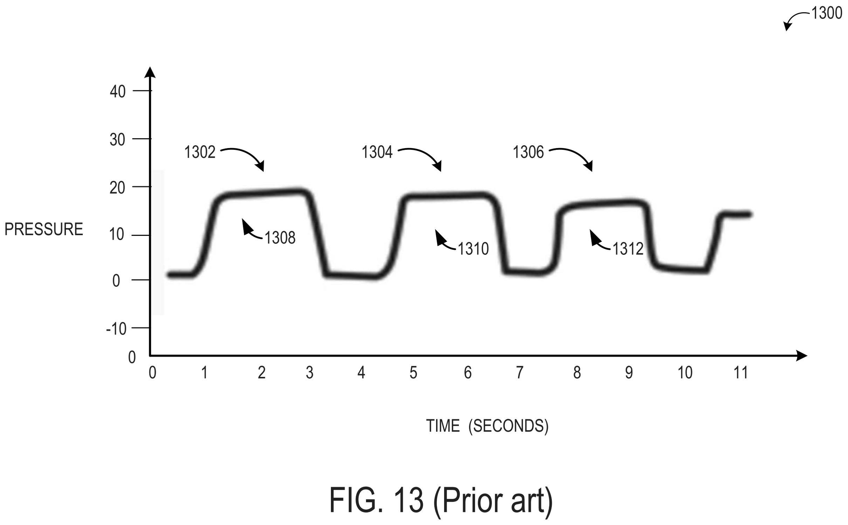

For example, FIGS. 13-15 show capnography data of patients suffering from various conditions, where the conditions may be at least partially diagnosed by a shape of the waveforms. FIG. 13 shows a first abnormal capnography plot 1300 of a patient, where the expiratory plateaus of waveforms of the patient are trending down. That is, a first expiratory plateau 1308 of a first waveform 1302 has a first height corresponding to a first pressure; a second expiratory plateau 1310 of a second waveform 1304 has a second height corresponding to a second pressure that is less than the first pressure; a third expiratory plateau 1312 of a third waveform 1306 has a third height corresponding to a third pressure that is less than the second pressure. This pattern of waveforms may be typically indicative of shock in the patient.

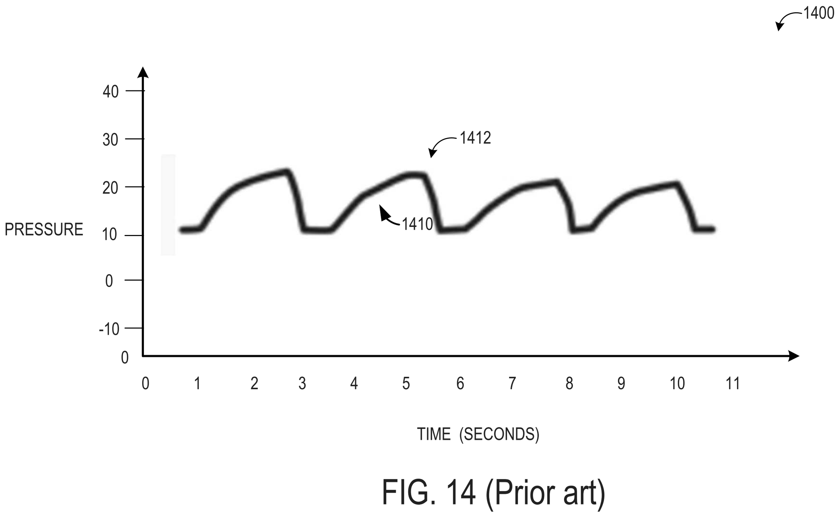

FIG. 14 shows a second abnormal capnography plot 1400 of a patient, where the expiratory instrokes 1410 of the waveforms are prolonged and the expiratory plateaus 1412 are shortened. This pattern of waveforms may be typically indicative of hypoxia due to asthma of the patient.

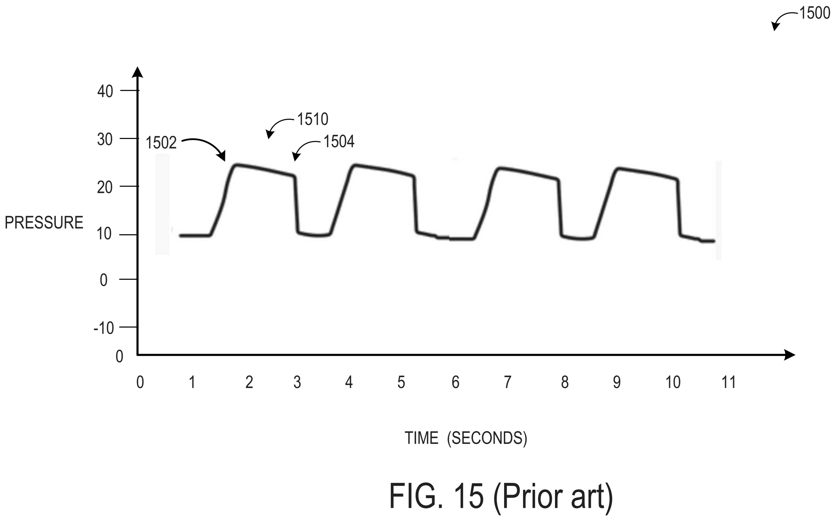

FIG. 15 shows a third abnormal capnography plot 1500 of a patient, where the expiratory plateaus of the waveforms trend down. That is, a greater pressure may be measured at a beginning 1502 of each expiratory plateau 1510 than at an end 1504 of the expiratory plateau 1510. This pattern of waveforms may be typically indicative of emphysema or leaking alveoli in a pneumothorax of the patient. Thus, a shape of a waveform in capnography data acquired from a patient may indicate a condition of the patient.

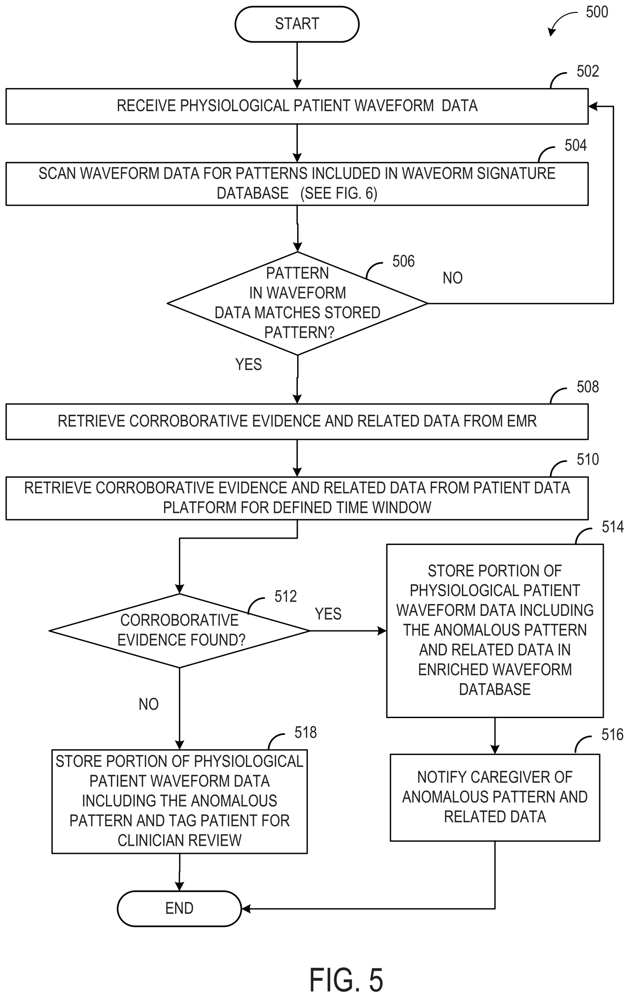

Turning now to FIG. 5, an exemplary method 500 is shown for detecting and notifying anomalous patterns in waveform data, such as the anomalous waveform patterns shown in FIGS. 13-15. Method 500 may be performed by a waveform anomaly detection system, such as waveform anomaly detection system 360 of FIG. 3. In an embodiment, operations of method 500 and the other methods described herein may be stored in non-transitory memory of the waveform anomaly detection system and executed by a processor of a cloud based processing module (e.g., cloud data processing module 342 of server 340 of FIG. 3).

Method 500 begins at 502, where method 500 includes receiving physiological patient waveform data of a patient. In various embodiments, the physiological patient waveform data may be acquired by a patient monitor (EKG, capnography system, etc.) positioned on a patient of a healthcare system, such as patient monitor 110. In other embodiments, the physiological patient waveform data may be generated by a different device.

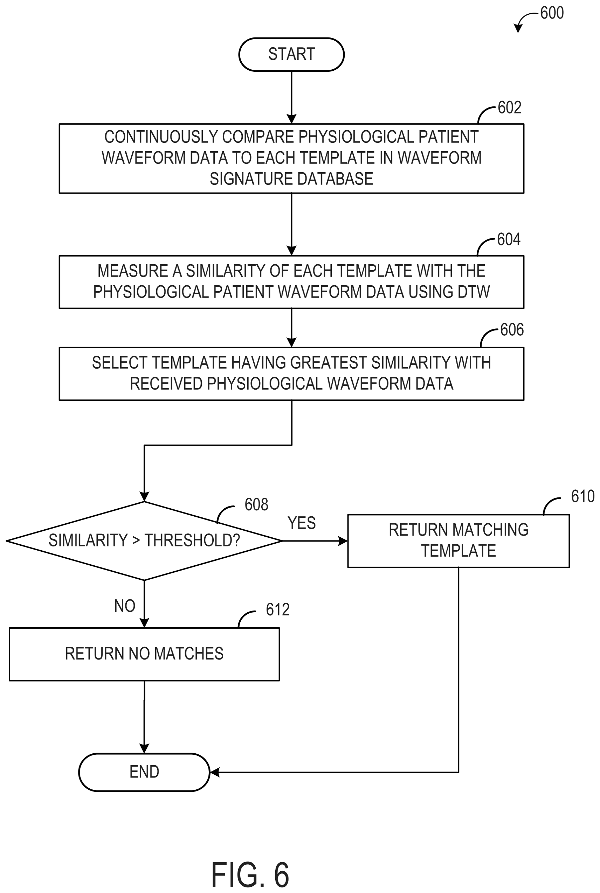

At 504, method 500 includes scanning the received physiological patient waveform data for patterns included in a waveform signature database (e.g., waveform signature database 364 of FIG. 3). The patterns may include anomalous patterns, such as the anomalous patterns shown in FIGS. 13-15. Scanning the received physiological patient waveform data for the anomalous patterns includes comparing portions of the received physiological patient waveform data with each pattern template included in the waveform signature database. Each pattern template may be generated from a pattern of a known anomaly found in medical literature. Scanning the physiological patient waveform data for anomalous patterns is described in greater detail in reference to FIG. 6.

At 506, method 500 includes determining whether a first pattern in the received physiological patient waveform data matches with a second pattern template included in the waveform signature database, in accordance with method 600 of FIG. 6. If no matches are detected at 506, method 500 proceeds back to 502, where the physiological patient waveform data continues to be received and scanned. If at 506 a match is detected, method 500 proceeds to 508.

At 508, method 500 includes retrieving corroborative evidence and related data from an EMR (e.g., EMR 350). The corroborative evidence may include, for example, demographic data of the patient; one or more lab reports of the patient; one or more medications prescribed to the patient; historical data of the patient; clinical notes associated with the patient; and/or other physiological parameter data of the patient. In various examples, the corroborative evidence may be retrieved using a rules-based model, such as a decision tree model created by human experts. The decision tree model may take data associated with the matching pattern as input, and search for relevant clinical data of the patient by applying one or more rules of the decision tree model.

At 510, method 500 includes retrieving corroborative evidence and related data from one or more patient data platforms on which data of the patient is stored. For example, the one or more patient data platforms may include other time-series data collected from the patient, or different patient data than the patient data included in the EMR. The corroborative evidence and related data may be retrieved based on a time window defined around a time of the detected anomalous waveform. For example, the time window may be within five hours of the time that the detected anomalous waveform was detected. The corroborative evidence and related data may be retrieved from the one or more patient data platforms by the decision tree model used at step 508 for the EMR data, or a different model.

As an example of how the corroborative evidence and related data may be retrieved, the physiological patient waveform data may include capnography time-series data, and a pattern of the capnography time-series data may be matched with a pattern in the waveform signature database associated with an airway obstruction, as in FIG. 14. As a result of the match, the decision tree model may retrieve a list of clinical data to be checked from a lookup table stored in a memory of the waveform anomaly detection system. The list of clinical data to be checked may include, for example, a cardiac output of the patient; a respiratory rate of the patient; a level of PCO2; a list of medications relevant to the airway obstruction; whether the patient suffers from conditions such as asthma, diabetes, etc.; and so on. For each element of clinical data on the list of clinical data, the decision tree model may look up, in a second lookup table stored in the memory of the waveform anomaly detection system, one or more data sources where the element of clinical data may be found. For example, the second lookup table may store a first location in an EMR where family history of asthma or diabetes may be checked; the second lookup table may store a second location in the EMR where prescribed medications of the patient may be retrieved; the second lookup table may store a connection data for connecting to a different patient monitor currently monitoring a blood pressure of the patient; and so on. The decision tree model may retrieve the elements of clinical data included in the first lookup table from respective data sources accessed from the second lookup table.

Further, in some examples, the decision tree model may include additional layers of logic or algorithms for obtaining additional clinical patient data, based on results retrieved from the list of clinical data. For example, the waveform anomaly detection system may determine that the patient is a diabetic, and as a result, the waveform anomaly detection system may retrieve clinical data of the patient related to diabetes (e.g., and not relating directly to the airway obstruction).

At 512, method 500 includes determining whether sufficient corroborative evidence was found to verify the matching anomalous waveform pattern. If at 512 sufficient evidence is found, method 500 proceeds to 514, where method 500 includes storing a portion of the physiological patient waveform data including the matching anomalous waveform pattern and the related patient data acquired at 508 and 510 in an enriched waveform database (e.g., enriched waveform database 366 of FIG. 3). That is, the related clinical data of the patient and the portion of the physiological patient waveform data may be stored in the memory such that when the portion of the physiological patient waveform data is retrieved from the memory, the retrieved clinical data is also retrieved from the memory. The portion of the physiological patient waveform data including the anomalous waveform pattern and the related clinical data are thus stored in an integrated fashion, such that the relationship between the portion of the physiological patient waveform data including the anomalous waveform pattern and the related clinical data is preserved.

At 516, method 500 includes notifying a caregiver (e.g., remote caregiver 390) of the patient of the anomalous waveform pattern. For example, the notification may be sent to a personal computing device of the caregiver, such as a smart phone (e.g., smart phone 140). When the caregiver opens the notification, or selects a control element in the notification, the portion of the physiological patient waveform data may be visualized on a display screen of the personal computing device. For example, the portion of the physiological patient waveform data may be visualized in an app (e.g., waveform review app 142) installed on the personal computing device. The integrated related clinical data of the patient may be concurrently displayed (e.g., superimposed) on the display screen and/or on the anomalous waveform pattern. The caregiver may interact with the displayed waveform pattern and/or the related patient data. For example, the caregiver may select points on the portion of the physiological patient waveform data and view data related to the points, open or close data panels, drill down for additional details, etc.

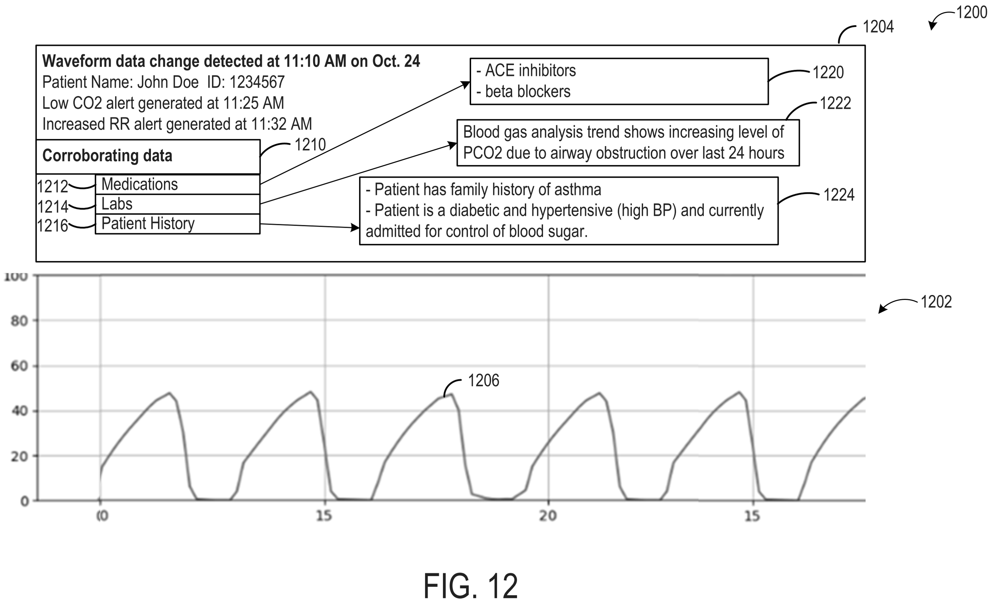

Referring briefly to FIG. 12, an exemplary UI 1200 of the waveform review app is shown, in which an exemplary portion of the physiological patient waveform data is visualized along with related clinical data in an integrated manner on a display device. UI 1200 includes a first data panel 1202 including a plot 1206 of the exemplary portion of the physiological patient waveform data. The exemplary portion shown in plot 1206 matches a pattern template for an obstructed airway included in the waveform signature database, which may be similar to the pattern shown in FIG. 14. As a result of the exemplary portion matching the pattern template for the obstructed airway, various clinical data relating to the obstructed airway may be retrieved from various EMRs and/or data platforms and displayed in a second display panel 1204.

Second display panel 1204 indicates a time that a change in the waveform data of the patient was detected, where the UI 1200 may have been prepared by the waveform anomaly detection system as a result of the change being detected. Second display panel 1204 further indicates that a low CO2 alert was generated and an increased respiratory rate alert was generated. Other general patient data may be displayed, such as patient identifying information.

The waveform review app may be configured to display in second display panel 1204 a menu 1210 listing categories of corroborating evidence of the obstructed airway retrieved from various hospital information systems and/or data platforms, where the corroborating evidence including elements of retrieved clinical data may be accessed directly from the menu. In the exemplary embodiment depicted in FIG. 12, menu 1210 includes a medications menu item 1212, a labs menu item 1214, and a patient history menu item 1216. Each of menu items 1212, 1214, and 1216 may be selectable, and when one of menu items 1212, 1214, and 1216 is selected, a limited list of elements of the retrieved clinical data may be displayed, where each element in the limited list is selectable to launch a display panel displaying additional information of the retrieved clinical data.

For example, the caregiver may select medications menu item 1212, and in response, a medications data panel 1220 may be displayed that shows a limited list of classes of medications that the patient is on (e.g., angiotensin-converting enzyme (ACE) inhibitors, beta blockers). Each class of medications may be selectable. The caregiver may select beta blockers, and in response, a list of specific medications may be displayed. The caregiver may select labs menu item 1214, and a labs data panel 1222 may be displayed that indicates that a level of PCO2 has been trending up. The caregiver may select patient history menu item 1216, and a patient history data panel 1224 may be displayed that indicates that the patient has a history of asthma and is a diabetic, and that the patient has been admitted for control of blood sugar. Thus, when viewing UI 1200 on the waveform review app, the caregiver may see a snapshot of a plurality of patient data relevant to monitoring or diagnosing the patient on a same screen as plot 1206, without having to connect to or log into the hospital information systems and/or data platforms. That is, the waveform data shown in first data panel 1202 and the related clinical data shown in second display panel 1204, medications menu item 1212, labs menu item 1214, and patient history menu item 1216 may be displayed even if the one or more EMRs and/or data platforms are not accessible over a network or in an unlaunched state. The related clinical data may be organized hierarchically, such that the caregiver may view the related clinical data by navigating successive levels of menu items.

Returning to method 500, if at 512 it is determined that sufficient corroborative evidence is not found to notify the caregiver, method 500 proceeds to 518, where method 500 includes storing the portion of the physiological patient waveform data including the anomalous waveform pattern without any integrated related patient data, and tagging the patient for clinician review. When the patient is tagged for clinician review, the caregiver may view the portion of the physiological patient waveform data including the anomalous waveform pattern at a subsequent time when reviewing the patient case. In some examples, the portion of the physiological patient waveform data may be discarded. Method 500 may end after 518 or 516.

Turning now to FIG. 6, an exemplary method 600 is shown for detecting anomalous waveforms in a set of physiological patient waveform data. Method 600 may be performed as part of method 500 of FIG. 5 described above.

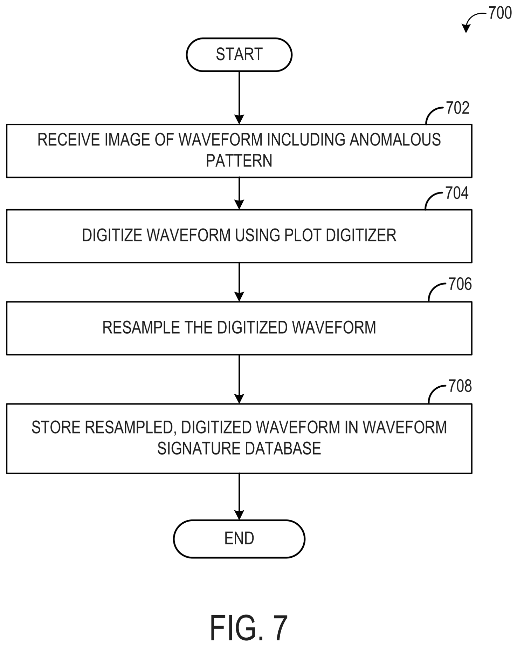

Method 600 begins at 602, where the method includes continuously comparing the physiological patient waveform data to a plurality of stored templates of waveform patterns having different shapes. Each stored template may be generated from an example of an anomaly included in standard medical literature that has been validated and is firmly established in clinical practice. The plurality of stored templates may be stored in a waveform signature database, such as waveform signature database 364 of FIG. 3. The waveform signature database may be populated with template patterns in a manual, semi-automated, or automated manner, as described below in reference to FIG. 7.

At 604, method 600 includes measuring a similarity of each template pattern with the physiological patient waveform data. The similarity between each template pattern and the physiological patient waveform data may be measured using one or more conventional techniques known in the art. In one example, the similarity may be measured using dynamic time warping (DTW). DTW is a method for measuring a similarity between two plots or curves representing time-series data where the plots or curves may have different lengths. The method has wide application, and is conventionally used in template matching, such as isolated word speech recognition (whether two sections of speech represent the same word or not), gesture recognition, data mining, information retrieval and the like. Under DTW, a time axis of the physiological patient waveform data may be stretched or shrunk to determine a closest match between each template and the physiological patient waveform data, and to find a canonical "shape" to represent the physiological patient waveform data. Under this measurement, a template classified as similar to the physiological patient waveform data may not necessarily have similar values at each point in time, but may have similar shapes.

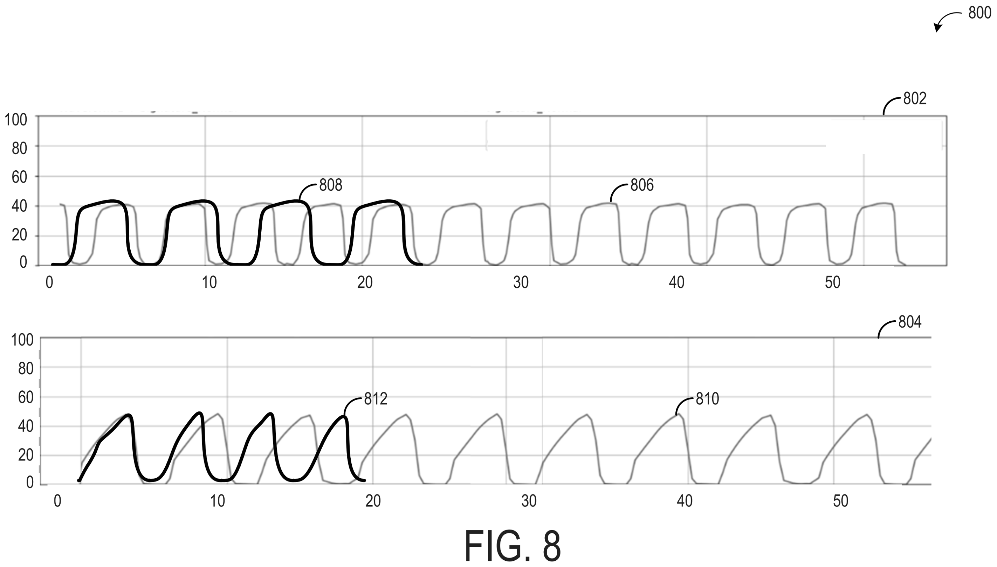

Referring briefly to FIG. 8, a waveform comparison diagram 800 shows a first capnography graph 802 and a second capnography graph 804. First capnography graph 802 includes a first plot 806, which may be a portion of continuous physiological patient waveform data acquired from a first patient via a patient monitor, such as patient monitor 110. First capnography graph 802 also includes a second plot 808, which may be a first template pattern included in the waveform signature database. The first template pattern may be a normal pattern associated with a healthy subject. In first capnography graph 802, first plot 806 may show the normal pattern associated with the healthy subject. However, first plot 806 does not exactly match second plot 808, because a timing of each capnographic waveform of plot 808 is longer than each capnographic waveform of plot 806.

Second capnography graph 804 includes a third plot 810, which may be a portion of continuous physiological patient waveform data acquired from a second patient via a similar patient monitor. Second capnography graph 804 also includes a fourth plot 812, which may be a second template pattern included in the waveform signature database. The second template pattern may be an anomalous pattern associated with a bronchospasm or asthma. In second capnography graph 804, third plot 810 may show the anomalous pattern associated with the bronchospasm or asthma. However, third plot 810 does not exactly match fourth plot 812, because a timing of each capnographic waveform of plot 812 is shorter than each capnographic waveform of plot 810.

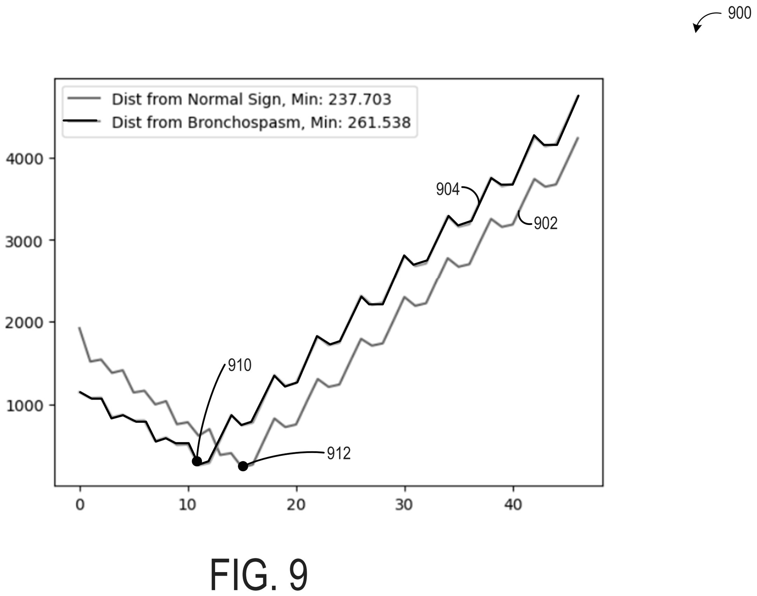

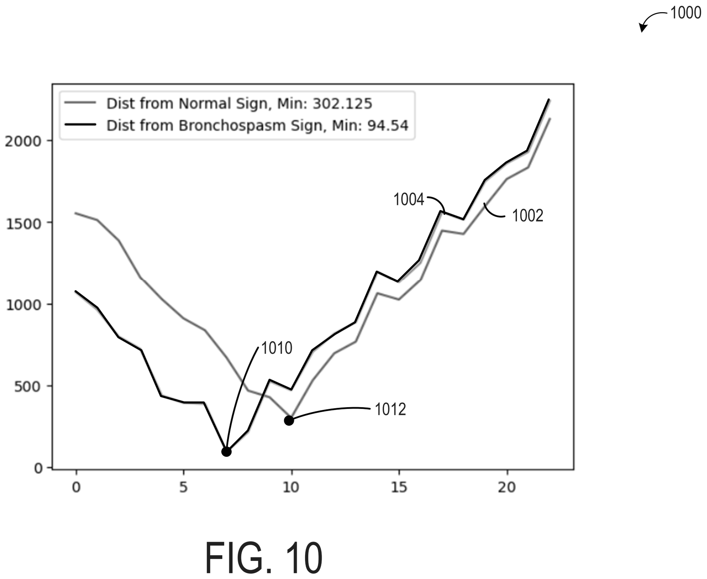

Returning to method 600, at 606, method 600 includes selecting a template having a greatest similarity with the physiological patient waveform data. The similarity may be determined using various methods. In one example, the template may be selected based on a minimum DTW distance of the physiological patient waveform data with the template pattern. An example of the calculation of the similarity of waveform data and template patterns shown in FIG. 8 is shown in FIGS. 9 and 10.

Referring briefly to FIG. 9, a first similarity graph 900 shows a first distance plot 902 and a second distance plot 904. From FIG. 8, the first physiological waveform acquired shown by first patient plot 806 neither matches exactly point to point with first template pattern plot 808 nor with the second template pattern plot 812. As a result, a DTW distance of a portion of the first physiological waveform shown by plot 806 from both the templates (plots 808 and 812) may be calculated. First distance plot 902 shows a first point-by-point DTW distance calculation between plot 806 and plot 808, which represents the first template pattern associated with a healthy patient included in the waveform signature database. Second distance plot 904 shows a second point-by-point DTW distance calculation between plot 806 and fourth plot 812, which represents the second template pattern included in the waveform signature database associated with a patient with a bronchospasm or asthma. The least DTW distance attained by template plot 808 with respect to the first patient physiological waveform is marked in FIG. 9 as a point 912. The least DTW distance attained by template plot 812 with respect to first patient physiological waveform is marked in FIG. 9 as a point 910. Point 912 is lower in first similarity graph 900 than 910, indicating that first patient physiological waveform is more similar to plot 808, which represents the normal pattern associated with a healthy subject.

FIG. 10 shows a second similarity graph 1000, including a first distance plot 1002 and a second distance plot 1004. From FIG. 8, the second physiological waveform acquired shown by second patient plot 810 neither matches exactly point to point with first template pattern plot 808 nor with the second template pattern plot 812. As a result, a DTW distance of a portion of the second physiological waveform shown by plot 810 from both the templates (plots 808 and 812) may be calculated. First distance plot 1002 shows a first point-by-point DTW distance calculation between plot 810 and plot 808, which represents the first template pattern associated with a healthy patient included in the waveform signature database. Second distance plot 1004 shows a second point-by-point DTW distance calculation between plot 810 and fourth plot 812, which represents the second template pattern included in the waveform signature database associated with a patient with a bronchospasm or asthma. The least DTW distance attained by template 808 with respect to the second patient physiological waveform is marked in FIG. 10 as a point 1012. The least DTW distance attained by template 812 with respect to the second patient physiological waveform is marked in FIG. 10 as a point 1010. Point 1010 is lower than point 1012 in second similarity graph 1000, indicating that second patient physiological waveform is more similar to plot 812, which represents the second template pattern included in the waveform signature database associated with a patient with a bronchospasm or asthma.

In various embodiments, the similarity algorithm may be applied to a plurality of template patterns stored in the waveform signature database, and a template pattern with a smallest distance calculation in accordance with the similarity algorithm may be selected as the most similar template pattern. In other embodiments, a different similarity metric or algorithm may be used to measure the similarity of physiological patient waveform data and template patterns of the waveform signature database.

Returning to method 600, at 608, the similarity of the selected template may be compared to a threshold similarity. If the similarity of the selected template is greater than the threshold similarity, method 600 proceeds to 610, where method 600 includes returning a matching template. Alternatively, if at 608 the similarity of the selected template is not greater than the threshold similarity, method 600 proceeds to 612, where method 600 includes returning no template matches.

Turning now to FIG. 7, an exemplary method 700 is shown for populating a waveform signature database such as waveform signature database 364 with a template pattern representing an anomalous waveform, which may be used in the execution of methods 500 and 600 of FIGS. 5 and 6, respectively. Method 700 may be performed by a processor of a waveform anomaly detection system such as waveform anomaly detection system 360 in a semi-autonomous manner, meaning that one or more steps of method 700 may rely on human intervention by an expert. Method 700 may be performed on a plurality of images of anomalous waveforms, in an iterative fashion, to populate the waveform signature database.

Method 700 begins at 702, where the method includes receiving an image of a waveform including an anomalous pattern, which may be selected from medical literature by a human expert. The selected image may be validated and firmly established in clinical practice. For example, reference medical literature may include medical text books used in clinical care focusing on basic physiology, vascular surgery, clinical aspects of waveform data, arterial waveform pattern derangement with advancing age, CO2 waveform pattern abnormality, echocardiography, including abnormal ECG waveform patterns, and others. The reference medical literature may also include medical journal publications, such as the British Medical Journal, National Institute of Health, Cambridge University Press, Sciencedirect, American heart association journals, and similar such scientific database platforms with access to peer reviewed medical publications and scholarly literature. In some examples, references to a text or a journal citing may be included as footnotes in a respective waveform anomaly template when the template is stored in the waveform anomaly database, to validate the credibility and authenticity of the information. Additionally, the waveform templates cited from various texts, journals and publications would be periodically reviewed and updated to ensure the information in the database does not become outdated or obsolete, and that new research outcomes published in journals, publications and clinical text books get periodically updated.

At 704, method 700 includes digitizing the waveform of the received image, using a plot digitizer, in accordance with various techniques known in the art. At 706, method 700 may include resampling the digitized waveform. Resampling may be achieved by first interpolating the waveform into a continuous graph, and then extracting equally spaced discrete samples from this graph. The resampling serves two purposes: a) removing noise that manual digitization would have introduced, and b) creating a consistent frequency waveform template for storage and similarity comparison. During manual digitization from an image, some data points may be slightly misplaced from their desired position. With interpolation before resampling, this noise may be suppressed to quite an extent. Manual digitization may not lead to equally spaced datapoints, and such a waveform may not be ideal for running similarity algorithms and storing templates in database. The similarity algorithms (including machine learning based algorithms) may expect the template waveform to be of a same frequency as the patient physiological waveform being streamed. Otherwise, a substantial amount of preprocessing may be involved when a similarity algorithm is called. Therefore, templates may be stored with a standard acceptable sampling rate. Additionally, storage and visualization of these templates may be much easier when sampled at a standard sampling rate.

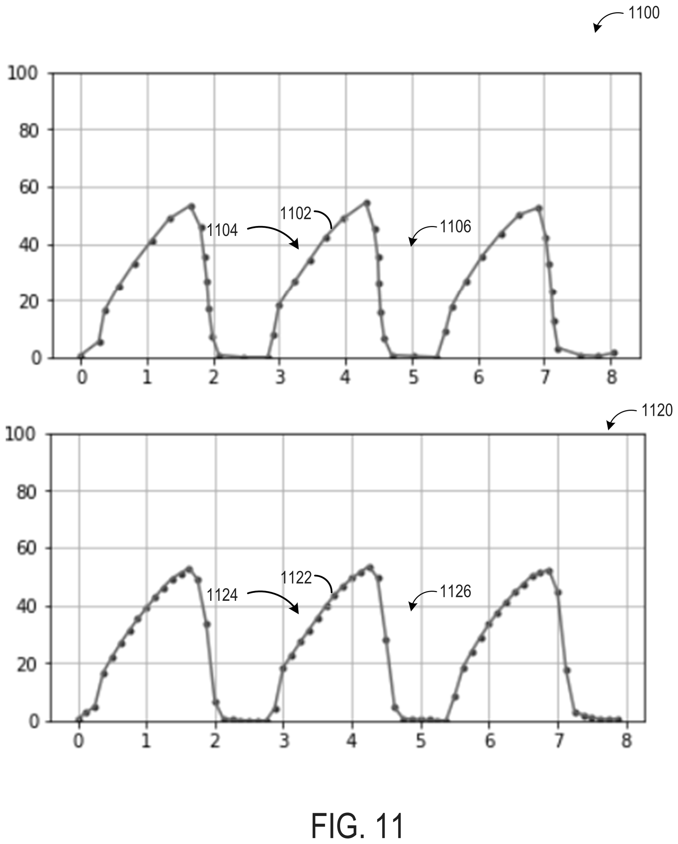

An example of a resampled waveform is shown in FIG. 11. Turning briefly to FIG. 11, it shows a first graph 1100 and a second graph 1120 of a bronchospasm template waveform. First graph 1100 includes a plot 1102 of the waveform, prior to interpolation and sampling, where plot 1102 may have been manually digitized from an image of the bronchospasm waveform in a medical text. An amplitude of the waveform is shown on the y axis, and time is shown on the x axis. Plot 1102 includes various repetitions of the waveform. During each repetition, the sample points from the manual digitization are equally spaced along plot 1102. However, the sample points are not equally spaced in time. For example, during a first portion 1104 of a waveform, there are three sample points between time t=3 and time t=4. However, during a second portion 1106 of the waveform, there are six sample points between time t=4 and time t=5. As a result, calculating a point-by-point similarity of plot 1102 with a waveform acquired from a patient may not be feasible.

Second graph 1120 includes a plot 1122 of the bronchospasm template waveform after interpolation and sampling. As with plot 1102, plot 1122 includes various repetitions of the waveform. In contrast with plot 1102, during each repetition, the sample points from the manual digitization are not equally spaced along plot 1102, rather, the sample points are equally spaced in time, as would be expected in a waveform acquired of a patient. During a first portion 1124 of a waveform, there are seven sample points between time t=3 and time t=4, and during a second portion 1126 of the waveform, there are seven sample points between time t=4 and time t=5. As a result, calculating a point-by-point similarity of plot 1102 with a waveform acquired from a patient may be facilitated.

Returning to method 700, at 708, method 700 includes storing the resampled, digitized waveform in the waveform signature database, and method 700 ends.

In this way, the waveform anomaly detection system provides an automated way to proactively identify anomalous portions of physiological patient waveform data and bring them to a clinician's attention, enriched with additional corroborative evidence. In contrast with current practice, clinicians may not have to rely on device-triggered alarm events based on deviations detected in the numerical parameter data, which may not detect anomalous patterns of interest in the physiological patient waveform data, or sort through huge amounts of time-series patient physiological data collected over a patient's stay in a care unit. By proactively identifying the anomalous portions, adverse clinical events may be prevented, thereby improving a quality of patient care and patient outcomes.

Unlike other pattern detection systems and methods, the waveform anomaly detection system described herein relies on automatically determining a similarity of the physiological patient waveform data with specific waveform patterns (e.g., without relying on a clinician to input a waveform or manually compare waveforms), where the patterns are taken from standard medical literature that has been validated over time and are widely accepted as accurate and firmly established in clinical practice. Unlike other systems that rely on a caregiver selecting a portion of the physiological patient waveform data to analyze, the waveform anomaly detection system performs a continuous comparison of moving windows of physiological patient waveform data against the stored waveform templates in real time, without human involvement.

Even if an alert is not triggered based on deviations in numerical parameter data, anomalous patterns may still be detected by the disclosed waveform anomaly detection system. Because the waveform anomaly detection system does not rely on the numerical parameter data, but rather compares shapes of waveforms generated in physiological patient data with the stored template patterns, the waveform anomaly detection system may represent a second, independent system for detecting abnormal conditions of a patient that may be used side by side with existing patient monitoring systems. Additionally, the pattern matching algorithm described herein may detect and flag worsening parameter trends before parameter limit alerts are triggered.

Further, when anomalies are detected in the physiological patient waveform data, the disclosed system searches for and retrieves clinical information related to the anomalous pattern matched to the physiological patient waveform data, such as related medical history, current medications being taken for related conditions, results of lab tests that may relate to the anomalous patterns, etc. The related clinical information is integrated into and stored with the physiological patient waveform data, such that a relationship between the related clinical information and the waveform data is preserved. When the physiological patient waveform data is viewed by a clinician, for example, as a result of being notified by the disclosed system, the related clinical information may be displayed to the clinician along with the waveform data in an integrated display. The integrated display may include information displayed at various levels, where the clinician may select a first element of related clinical data, and a data panel may be displayed (e.g., popped up) that shows a second element of related clinical data.

For example, the waveform anomaly detection system may include a model, such as a decision tree model, that takes as input a specification of an anomalous waveform, such as those depicted in FIGS. 14 and 15. The model may apply one or more rules to the specification of the anomalous waveform to determine types of clinical data to retrieve, and where/how to retrieve the data. The model may apply a second set of rules to determine whether to retrieve additional data (e.g., additional lab tests, previous medications, etc.) from the hospital databases, EMRs, and data platforms. The clinical data may then be stored with the waveform data, and presented in a multi-tiered format that may be generated, for example, by a smart phone app or application of the waveform anomaly detection system running on a personal computing device, where the clinician can select links to data or menu items to view additional information and "drill down" through the information viewed.

In other words, the retrieval of the clinical data is performed automatically at a first time, when the anomalous pattern is detected, without relying on the clinician to specify search parameters. The clinical data may be reviewed at a second, later time, when the data has been compiled, organized, and integrated into the waveform data. In some examples, the application may be configured to display in a GUI a preview of the related clinical data that can be reached directly from a menu, where the preview displays one or more limited sets of data that are selectable to launch a view or visualization of additional related clinical data.

As a result, the related clinical information may be displayed to the clinician without the clinician having to access or log into hospital databases, EMRs, or data platforms, which may not be available at the time. That is, the waveform data and related clinical information may be viewed even when one or more of the hospital databases, EMRs, or data platforms are in an unlaunched state. As a result, the related clinical information may be displayed to the clinician more rapidly and intuitively than by current methods, where the clinician would have to manually review raw patient physiological data to determine whether an anomaly may be present, and then manually search for related clinical information in disparate information systems and data platforms.

The technical effect of identifying anomalies in physiological patient waveform data by comparing portions of the physiological patient waveform data with a plurality of template patterns of anomalous waveforms stored in a database, retrieving clinical data related to an identified anomaly, and displaying the clinical data to a caregiver in an integrated display, is that the anomalies may be detected without relying on alerts or sifting through a large amount of waveform data manually. Additionally, the caregiver may review a larger amount of patient data related to a condition of the patient then would be feasible if the caregiver had to log into each data source and retrieve the related clinical data individually. A further technical effect is that the data may be presented to the caregiver even when hospital information systems are unavailable or inaccessible.

The disclosure also provides support for a waveform anomaly detection system, comprising: a processor, and a memory storing instructions that when executed, cause the processor to: receive physiological patient waveform data acquired via a sensor positioned on a body of a patient, detect an anomalous pattern in a portion of the physiological patient waveform data in real time as the physiological patient waveform data is received, retrieve clinical data relating to the anomalous pattern of the patient from one or more electronic medical record (EMR) systems, store the retrieved clinical data together with the portion of the physiological patient waveform data in the memory, and display the portion of the physiological patient waveform data having the anomalous pattern on a display device together with the retrieved clinical data and/or a link to the retrieved clinical data, wherein detecting the anomalous pattern in the portion of the physiological patient waveform data further comprises comparing the portion with a plurality of stored templates of anomalous patterns of waveform data stored in a waveform signature database in the memory, the templates generated from examples of anomalies included in standard medical literature that are validated and firmly established in clinical practice. In a first example of the system, the retrieved clinical data together and the portion of the physiological patient waveform data are stored in the memory such that when the physiological patient waveform data is retrieved from the memory, the retrieved clinical data is also retrieved from the memory. In a second example of the system, optionally including the first example, further instructions are stored in the memory that when executed, cause the processor to send a notification to a personal computing device of a caregiver of the patient, and the portion of the physiological patient waveform data having the anomalous pattern on a display device and the retrieved clinical data and/or the link to the retrieved clinical data is displayed in response to the caregiver opening the notification on the personal computing device. In a third example of the system, optionally including one or both of the first and second examples, the personal computing device is a smart phone of the caregiver, and the portion of the physiological patient waveform data having the anomalous pattern on a display device and the retrieved clinical data and/or the link to the retrieved clinical data is displayed via an app of the waveform anomaly detection system installed on the smart phone. In a fourth example of the system, optionally including one or more or each of the first through third examples,: the app is configured to display on the display device a menu listing categories of the retrieved clinical data viewable on the display device, and the app is additionally configured to display on the display device a plurality of elements of the retrieved clinical data that can be reached directly from the menu, wherein in response to the caregiver selecting a menu item of the menu, a limited list of elements of the retrieved clinical data is displayed, each element in the limited list being selectable to launch a display panel displaying additional information of the retrieved clinical data, and wherein the additional information of the retrieved clinical data is displayed while the one or more EMR systems are in an unlaunched state. In a fifth example of the system, optionally including one or more or each of the first through fourth examples, the physiological patient waveform data is continuously compared to the plurality of stored templates of anomalous patterns of waveform data in real time, and the portion of the physiological patient waveform data is not selected by a person. In a sixth example of the system, optionally including one or more or each of the first through fifth examples, the waveform anomaly detection system is located in a cloud, and the waveform signature database is stored in the cloud. In a seventh example of the system, optionally including one or more or each of the first through sixth examples, the physiological patient waveform data includes one of electrocardiogram (ECG) waveform data, pleth waveform data acquired via a pulse oximeter, arterial waveform data, and end-tidal carbon dioxide (ETCO2) waveform data acquired via a capnography system, and the clinical data relating to the anomalous pattern of the patient retrieved from the one or more EMR systems includes one of demographic data of the patient, a lab report of the patient, a medication prescribed to the patient, historical data of the patient, clinical notes associated with the patient, and other physiological parameter data of the patient. In an eighth example of the system, optionally including one or more or each of the first through seventh examples, further instructions are stored in the memory that when executed, cause the processor to retrieve clinical data of the patient corresponding to a time window of the anomalous pattern of the patient from a medical data platform storing physiological patient data of the patient. In a ninth example of the system, optionally including one or more or each of the first through eighth examples, further instructions are stored in the memory that when executed, cause the processor to: receive an image of a waveform selected from the medical literature, digitize the waveform using a plot digitizer, resample the digitized waveform, and store the resampled waveform in the waveform signature database. In a tenth example of the system, optionally including one or more or each of the first through ninth examples, further instructions are stored in the memory that when executed, cause the processor to: in response to receiving a submission of a template of an anomalous pattern from a user of the waveform anomaly detection system, the template not matching any existing templates in the waveform signature database, add the template to the plurality of stored templates of anomalous patterns of waveform data stored in the waveform signature database. In a eleventh example of the system, optionally including one or more or each of the first through tenth examples, further instructions are stored in the memory that when executed, cause the processor to: continuously compare the received physiological patient waveform data to each template in the waveform signature database, use dynamic time warping (DTW) to measure a similarity of each template with the received physiological patient waveform data, and select a template having a greatest similarity with the received physiological patient waveform data. In a twelfth example of the system, optionally including one or more or each of the first through eleventh examples, the anomalous pattern detected in the portion of the physiological patient waveform data does not cause an alert to be generated by software algorithms not included in the waveform anomaly detection system. In a thirteenth example of the system, optionally including one or more or each of the first through twelfth examples, the anomalous pattern is not detected by a machine learning (ML) algorithm.

The disclosure also provides support for a method for a waveform anomaly detection system, the method comprising: receiving physiological patient waveform data acquired via a sensor positioned on a body of a patient, detecting an anomalous pattern in a portion of the physiological patient waveform data in real time as the physiological patient waveform data is received, retrieving clinical data relating to the anomalous pattern of the patient from one or more electronic medical record (EMR) systems, storing the retrieved clinical data together with the portion of the physiological patient waveform data in a memory of the waveform anomaly detection system, and displaying the portion of the physiological patient waveform data having the anomalous pattern on a display device together with the retrieved clinical data and/or a link to the retrieved clinical data, wherein detecting the anomalous pattern in the portion of the physiological patient waveform data further comprises comparing the portion with a plurality of stored templates of anomalous patterns of waveform data stored in a waveform signature database in the memory, the templates generated from examples of anomalies included in standard medical literature that are validated and firmly established in clinical practice. In a first example of the method, the method further comprises: sending a notification to a device of a caregiver of the patient, that when opened, displays the portion of the physiological patient waveform data having the anomalous pattern on a display device and the retrieved clinical data and/or the link to the retrieved clinical data on the device. In a second example of the method, optionally including the first example, the method further comprises: retrieving clinical data of the patient corresponding to a time window of the anomalous pattern of the patient from a medical data platform storing physiological patient data of the patient. In a third example of the method, optionally including one or both of the first and second examples, detecting the anomalous pattern in the portion of the physiological patient waveform data further comprises: continuously comparing the received physiological patient waveform data to each template in the waveform signature database, measuring a similarity of each template with the received physiological patient waveform data using dynamic time warping (DTW), and selecting a template having a greatest similarity with the received physiological patient waveform data.

The disclosure also provides support for a method for populating a database of a waveform anomaly detection system with a plurality of examples of anomalous physiological patient waveform data, the method comprising: receiving an image of anomalous physiological patient waveform data taken from established medical literature, digitizing the patient waveform data using a plot digitizer, resampling the digitized patient waveform data, and storing the resampled waveform data in a database. In a first example of the method, the image is an image of an anomalous waveform encountered by a user of the waveform anomaly detection system that is not taken from the established medical literature.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative and should not be construed to be limiting in any manner.

## Claims

1. A waveform anomaly detection system (360), comprising:
a processor, and a memory storing instructions that when executed, cause the processor to:
receive physiological patient waveform data acquired via a sensor (370) positioned on a body of a patient (102);
detect an anomalous pattern (1300, 1400, 1500) in a portion of the physiological patient waveform data in real time as the physiological patient waveform data is received;
retrieve clinical data relating to the anomalous pattern (1300, 1400, 1500) of the patient (102) from one or more electronic medical record (EMR) systems (350);
store the retrieved clinical data together with the portion of the physiological patient waveform data in the memory; and
display the portion of the physiological patient waveform data having the anomalous pattern (1300, 1400, 1500) on a display device (140) together with the retrieved clinical data and/or a link to the retrieved clinical data;
wherein detecting the anomalous pattern (1300, 1400, 1500) in the portion of the physiological patient waveform data further comprises comparing the portion with a plurality of stored templates (808, 812) of anomalous patterns (1300, 1400, 1500) of waveform data stored in a waveform signature database (364) in the memory, the templates (808, 812) generated from examples of anomalies included in standard medical literature that are validated and firmly established in clinical practice.

2. The waveform anomaly detection system (360) of claim 1, wherein the retrieved clinical data together and the portion of the physiological patient waveform data are stored in the memory such that when the physiological patient waveform data is retrieved from the memory, the retrieved clinical data is also retrieved from the memory.

3. The waveform anomaly detection system (360) of claim 1, wherein further instructions are stored in the memory that when executed, cause the processor to send a notification to a personal computing device (140) of a caregiver of the patient (102), and the portion of the physiological patient waveform data having the anomalous pattern (1300, 1400, 1500) and the retrieved clinical data and/or the link to the retrieved clinical data is displayed in response to the caregiver opening the notification on the personal computing device (140).

4. The waveform anomaly detection system (360) of claim 3, wherein the personal computing device (140) is a smart phone (140) of the caregiver, and the portion of the physiological patient waveform data having the anomalous pattern and the retrieved clinical data and/or the link to the retrieved clinical data is displayed via an app (142) of the waveform anomaly detection system (360) installed on the smart phone (140).

5. The waveform anomaly detection system (360) of claim 4, wherein:
the app (142) is configured to display on the display device a menu (1210) listing categories (1212, 1214, 1216) of the retrieved clinical data viewable on the display device; and
the app (142) is additionally configured to display on the display device a plurality of elements (1220, 1222, 1224) of the retrieved clinical data that can be reached directly from the menu (1210);
wherein in response to the caregiver selecting a menu item (1212, 1214, 1216) of the menu (1210), a limited list of elements (1220, 1222, 1224) of the retrieved clinical data is displayed, each element in the limited list being selectable to launch a display panel displaying additional information of the retrieved clinical data; and
wherein the additional information of the retrieved clinical data is displayed while the one or more EMR systems (360) are in an unlaunched state.

6. The waveform anomaly detection system (360) of claim 1, wherein the physiological patient waveform data is continuously compared to the plurality of stored templates (818, 812) of anomalous patterns (1300, 1400, 1500) of waveform data in real time, and the portion of the physiological patient waveform data is not selected by a person.

7. The waveform anomaly detection system (360) of claim 1, wherein the waveform anomaly detection system (360) is located in a cloud, and the waveform signature database (364) is stored in the cloud.

8. The waveform anomaly detection system (360) of claim 1, wherein the physiological patient waveform data includes one of electrocardiogram (ECG) waveform data, pleth waveform data acquired via a pulse oximeter, arterial waveform data, and end-tidal carbon dioxide (ETCO2) waveform data acquired via a capnography system; and the clinical data relating to the anomalous pattern (1300, 1400, 1500) of the patient (102) retrieved from the one or more EMR systems includes one of demographic data of the patient (102), a lab report of the patient (102), a medication prescribed to the patient (102), historical data of the patient (102), clinical notes associated with the patient (102), and other physiological parameter data of the patient (102).

9. The waveform anomaly detection system (360) of claim 1, wherein further instructions are stored in the memory that when executed, cause the processor to retrieve clinical data of the patient (102) corresponding to a time window of the anomalous pattern (1300, 1400, 1500) of the patient (102) from a medical data platform (352) storing physiological patient data of the patient (102).

10. The waveform anomaly detection system (360) of claim 1, wherein further instructions are stored in the memory that when executed, cause the processor to:
receive an image of a waveform selected from the medical literature;
digitize the waveform using a plot digitizer;
resample the digitized waveform; and
store the resampled waveform in the waveform signature database (354).

11. The waveform anomaly detection system (360) of claim 1, wherein further instructions are stored in the memory that when executed, cause the processor to:
in response to receiving a submission of a template (802, 812) of an anomalous pattern from a user of the waveform anomaly detection system (360), the template (802, 812) not matching any existing templates (802, 812) in the waveform signature database (354), add the template (802, 812) to the plurality of stored templates (802, 812) of anomalous patterns of waveform data stored in the waveform signature database (354).

12. The waveform anomaly detection system (360) of claim 1, wherein further instructions are stored in the memory that when executed, cause the processor to:
continuously compare the received physiological patient waveform data to each template (802, 812) in the waveform signature database;
measure a similarity of each template (802, 812) with the received physiological patient waveform data; and
select a template (802, 812) having a greatest similarity with the received physiological patient waveform data.

13. The waveform anomaly detection system (360) of claim 1, wherein the anomalous pattern (1300, 1400, 1500) detected in the portion of the physiological patient waveform data does not cause an alert to be generated by software algorithms not included in the waveform anomaly detection system (360).

14. The waveform anomaly detection system (360) of claim 1, wherein the anomalous pattern (1300, 1400, 1500) is not detected by a machine learning (ML) algorithm.

15. A method (500) for a waveform anomaly detection system, the method comprising:
receiving physiological patient waveform data acquired via a sensor positioned on a body of a patient (502);
detecting an anomalous pattern in a portion of the physiological patient waveform data in real time as the physiological patient waveform data is received (504);
retrieving clinical data relating to the anomalous pattern of the patient from one or more electronic medical record (EMR) systems (508);
storing the retrieved clinical data together with the portion of the physiological patient waveform data in a memory of the waveform anomaly detection system; and
displaying the portion of the physiological patient waveform data having the anomalous pattern on a display device together with the retrieved clinical data and/or a link to the retrieved clinical data (514);
wherein detecting the anomalous pattern in the portion of the physiological patient waveform data further comprises comparing the portion with a plurality of stored templates of anomalous patterns of waveform data stored in a waveform signature database in the memory, the templates generated from examples of anomalies included in standard medical literature that are validated and firmly established in clinical practice.
